# EUROPÄISCHE PATENTSCHRIFT

(11) **EP 1 744 693 B1**
(45) Veröffentlichungstag und Bekanntmachung des Hinweises auf die Patenterteilung: **11.01.2017**
(21) Anmeldenummer: 05737400.1
(22) Anmeldetag: 22.04.2005
(51) Int. Cl.: A61B 17/88, A61F 2/30, G09B 23/30

(54) **DREIDIMENSIONALES MODELL EINES MENSCHLICHEN KINDERSCHÄDELS IN LEBENSGRÖSSE**
THREE-DIMENSIONAL LIFE SIZE MODEL OF A CHILD'S SKULL
MODELE EN TROIS DIMENSIONS D'UN CRANE D'ENFANT HUMAIN GRANDEUR NATURE

(30) Priorität: 23.04.2004 DE 102004020020
(43) Veröffentlichungstag der Anmeldung: 24.01.2007
(60) Teilanmeldung: 12169571.2
(73) Patentinhaber: IBB Technologie-Entwicklungs-Fonds GmbH & Co. KG (TEF)., 10719 Berlin (DE)
(72) Erfinder: HABERL, Hannes, 10719 Berlin (DE)
(74) Vertreter: Tegethoff, Sebastian
(86) Internationale Anmeldenummer: PCT/EP2005/004361
(87) Internationale Veröffentlichungsnummer: WO 2005/104061

(56) Entgegenhaltungen:
- EP-A- 0 720 835
- US-A- 3 009 265
- US-A- 5 156 777
- US-A- 5 397 361
- US-A- 6 112 109

## Beschreibung

Die Erfindung betrifft ein dreidimensionales Modell eines menschlichen Kinderschädels in Lebensgröße, ein Kit, umfassend mehrere solcher Modelle sowie Verfahren unter Verwendung des Modells.

Die Erforschung der Korrekturmöglichkeiten genetischer Störungen des Schädelwachstums auf zellulärer Ebene hat bisher nicht zu praktisch verwendbaren Ergebnissen geführt. Patienten mit prämaturen Schädelnahtsynostosen (Inzidenz 1:1000) sind deshalb weiterhin auf eine operative Korrektur angewiesen um ihr Aussehen zu verbessern oder das Risiko eines funktionellen Schadens zu senken.

Die schnelle Zunahme des Hirnvolumens in den ersten Lebensmonaten spricht für eine möglichst frühe Durchführung des notwendigen umfangreichen Eingriffes. Das im Sinne einer Risikoabwägung günstigste Operationsalter wird zwischen 4 und 12 Monaten angegeben. Der erforderliche Eingriff ist ausgedehnt und gemessen am Lebensalter mit einigen Risiken behaftet. Insbesondere der relativ hohe Blutverlust läßt eine Vereinfachung und Verkürzung der Operationszeit besonders wünschenswert erscheinen.

US 5,156,777 beschreibt ein Verfahren zum Herstellen einer implantierbaren Prothese für eine vorbestimmte Stelle an oder in einem Organ. Zunächst wird ein Implantatmodell angefertigt, das als Form für eine Negativform dient. Diese Negativform wird anschließend mit einem biologisch inerten, flexiblen und komprimierbaren Material, wie etwa Silikongummigefüllt, um die tatsächliche implantierbare Prothese zu bilden. Ein dreidimensionales lebensgroßes Kinderschädelmodell, welches sterilisierbar ist, wird nicht offenbart.

US 6,112,109 beschreibt ein Verfahren zum konstruktiven Modellieren von Artikeln, einschließlich Prothesen, bei denen Computertomographiedaten verwendet werden, um ein dreidimensionales Modell zu erstellen. Ein dreidimensionales Modell eines Kinderschädels, welches sterilisierbar ist, ist nicht offenbart.

In dem Patent US 3,009,265 geht es um ein Schädelmodell, welches aus mehreren Komponenten besteht, zum Einsatz in der Lehre oder in der Kunst. Es geht nicht um Planungskriterien bei der Erstellung von Schädelformen von Patienten und auch nicht um ein Kinderschädelmodell.

Das Patent US 5,397,361 beschäftigt sich mit Kranialplatten und somit mit Teilen des Hirnschädels, die modelliert und implantiert werden sollen. Es geht nicht um dreidimensionale Modelle von Kinderschädeln.

Operationstechnisch haben sich weltweit die von Paul Tessier (Tessier P. Total facial osteotomy. Crouzon's syndrome, Apert's syndrome: oxycephaly, scaphocephaly, turricephaly. Ann Chir Plast 1967; 12(4): 273-86) vorgeschlagenen Methoden der Knochenfragmentierung, - umformung und Reposition durchgesetzt. Operationstechnik und Zeitpunkt wurden in der Folgezeit von verschiedenen Autoren variiert (Renier D, Lajeunie E, Arnaud E, Marchac D. Management of craniosynostoses. Childs Nerv Syst 2000; 16(10-11):645-58). Im Laufe der letzten 30 Jahre konnte so eine gewisse Standardisierung des tradierten technischmethodischen Vorgehens beobachtet werden.

Einige wichtige und zeitraubende Aspekte des operativen Vorgehens haben allerdings bisher keine richtungsweisende Diskussion erfahren:
1. Die Kriterien für ein exaktes Form-"Ziel" fehlen. Die wichtigste und oft einzige Operationsindikation - die möglichst perfekte Annäherung an die fiktive "gesunde" Schädelform des Patienten - bleibt ohne Planungskriterien dem Formgefühl des Operateurs bzw. seinen willkürlichen ästhetischen Kriterien überlassen. Die Operationszeit kann durch immer wieder erforderliche Korrekturen im Sinne eines Herantastens an die optimale Form verlängert werden. Eine objektive Erfolgskontrolle ist wegen der individuellen Ausformung nahezu ausgeschlossen.
2. Formwahl und Realisierung im Bereich unterschiedlich stark gekrümmter Kalottenabschnitte, etwa im Stirnbereich, besitzen aufgrund der Materialsteifigkeit auch für den trainierten Operateur einen hohen Schwierigkeitsgrad.

Sie sind langwierig und zeigen nicht seiten in zumindest einer Ebene suboptimale Ergebnisse.

Dementsprechend war es Aufgabe der vorliegenden Erfindung, Zielkriterien für eine Behandlung von Cranialsynostosen bereitzustellen. Es war eine weitere Aufgabe der Erfindung, die operationstechnischen Probleme der Schädelumformung bei angeborenen oder erworbenen Schädeldeformitäten zu lösen.

Eine weitere Aufgabe der vorliegenden Erfindung war es, ein Instrumentarium zur Verbesserung und Vereinfachung operativer Schädelrekonstruktionen im ersten Lebensjahr bereitzustellen.

Diese Aufgaben werden gelöst durch ein dreidimensionales Modell eines menschlichen Kinderschädels in Lebensgröße, umfassend einen Stirnteil (Frontalmodul), einen Scheitelteil (Parietalmodul) und einen Hinterhauptteil (Occipitalmodul), dadurch gekennzeichnet, daß es sterilisierbar ist. Es ist vorgesehen, daß das erfindungsgemäße dreidimensionale Modell zusätzlich einen Basisteil (Basismodul) umfaßt, auf dem Stirnteil, Scheitelteil und Hinterhauptteil abnehmbar angeordnet sind.

Der Begriff "sterilisierbar", wie hierin verwendet, soll bedeuten, daß das Modell in der Lage ist, Temperaturen und Drücken und/oder Gaseinwirkungen zu widerstehen, wie sie üblicherweise beim Autoklavieren oder Sterilisieren von Operationsbesteck, medizinischem Gerät zum Einsatz am und im Patienten etc. verwendet werden, ohne daß das Modell durch einen solchen Sterilisierungsvorgang beschädigt, insbesondere deformiert würde. Bevorzugt ist das Modell aus einem entsprechenden hierfür geeigneten Material, bspw. Stahl, Titan oder Kunststoff.

In einer Ausführungsform ist vorgesehen, daß in dem Modell mindestens zwei Teile, ausgewählt aus Stirnteil, Scheitelteil und Hinterhauptteil, miteinander verbunden sind, wobei bevorzugt ist, daß der Scheitelteil und Hinterhauptteil miteinander zu einem Scheitel- und Hinterhauptteil (Parieto-Occipitalmodul) verbunden sind.

In einer Ausführungsform ist vorgesehen, daß das erfindungsgemäße dreidimensionale Modell einen durchschnittlichen Kinderschädel darstellt, wobei der Durchschnitt auf eine normal verteilte Population von Kindern bezogen ist, die 12-20 Wochen alt sind.

In einer anderen Ausführungsform ist vorgesehen, daß das erfindungsgemäße dreidimensionale Modell einen durchschnittlichen Kinderschädel darstellt, wobei der Durchschnitt auf eine normal verteilte Population von Kindern bezogen ist, die 24-32 Wochen alt sind.

Es wird bevorzugt, daß das erfindungsgemäße Modell hinsichtlich eines oder mehrerer der Merkmale, ausgewählt aus der Gruppe, umfassend horizontale Stirnwölbung, vertikale Stirnwölbung, Stirnbreite, frontonasaler Winkel, frontooccipitaler Durchmesser, biparietaler Durchmesser und horizontaler Krümmungsradius des Occipitalmoduls, einen durchschnittlichen Kinderschädel darstellt.

In einer Ausführungsform ist vorgesehen, daß das erfindungsgemäße dreidimensionale Modell hinsichtlich eines oder mehrerer der Merkmale, ausgewählt aus der Gruppe, umfassend horizontale Stirnwölbung, vertikale Stirnwölbung, Stirnbreite, frontonasaler Winkel, frontooccipitaler Durchmesser, biparietaler Durchmesser und horizontaler Krümmungsradius des Occipitalmoduls, auf einer Perzentilenkurve liegt, die ≤ 10% ist, bezogen auf eine normal verteilte Population von Kindern im Alter von 0-1 Jahren, wobei bevorzugt ist, daß es auf einer perzentilen Kurve liegt, die 3% ≤ x% ≤ 10%, bevorzugt 3% ist.

In einer anderen Ausführungsform ist vorgesehen, daß das erfindungsgemäße dreidimensionale Modell hinsichtlich eines oder mehrerer der Merkmale, ausgewählt aus der Gruppe, umfassend horizontale Stirnwölbung, vertikale Stirnwölbung, Stirnbreite, frontonasaler Winkel, frontooccipitaler Durchmesser, biparietaler Durchmesser, horizontaler Krümmungsradius des Occipitalmoduls, auf einer Perzentilenkurve liegt, die 10% < x% ≤ 30% ist, bezogen auf eine normal verteilte Population von Kindern im Alter von 0-1 Jahren.

In einer anderen Ausführungsform ist vorgesehen, daß das erfindungsgemäße dreidimensionale Modell hinsichtlich eines oder mehrerer der Merkmale, ausgewählt aus der Gruppe, umfassend horizontale Stirnwölbung, vertikale Stirnwölbung, Stirnbreite, frontonasaler Winkel, frontooccipitaler Durchmesser, biparietaler Durchmesser, horizontaler Krümmungsradius des Occipitalmoduls, auf einer Perzentilenkurve liegt, die 30% < x% ≤ 70% ist, bezogen auf eine normal verteilte Population von Kindern im Alter von 0-1 Jahren, wobei bevorzugt ist, daß es auf einer Perzentilenkurve liegt, die 45% ≤ x% ≤ 55% ist, bezogen auf eine normal verteilte Population von Kindern im Alter von 0-1 Jahren, wobei besonders bevorzugt ist, daß es auf einer Perzentilenkurve liegt, die 50% ist, bezogen auf eine normal verteilte Population von Kindern im Alter von 0-1 Jahren.

In einer Ausführungsform ist vorgesehen, daß das erfindungsgemäße dreidimensionale Modell hinsichtlich eines oder mehrerer der Merkmale, ausgewählt aus der Gruppe, umfassend horizontale Stirnwölbung, vertikale Stirnwölbung, Stirnbreite, frontonasaler Winkel, frontooccipitaler Durchmesser, biparietaler Durchmesser, horizontaler Krümmungsradius des Occipitalmoduls, auf einer Perzentilenkurve liegt, die 55% < x% ≤ 70% ist, bezogen auf eine normal verteilte Population von Kindern im Alter von 0-1 Jahren.

In einer anderen Ausführungsform ist vorgesehen, daß das erfindungsgemäße dreidimensionale Modell hinsichtlich eines oder mehrerer der Merkmale, ausgewählt aus der Gruppe, umfassend horizontale Stirnwölbung, vertikale Stirnwölbung, Stirnbreite, frontonasaler Winkel, frontooccipitaler Durchmesser, biparietaler Durchmesser, horizontaler Krümmungsradius des Occipitalmoduls, auf einer Perzentilenkurve liegt, die 70% < x% ≤ 90% ist, bezogen auf eine normal verteilte Population von Kindern im Alter von 0-1 Jahren.

In einer anderen Ausführungsform ist vorgesehen, daß das erfindungsgemäße dreidimensionale Modell hinsichtlich eines oder mehrerer der Merkmale, ausgewählt aus der Gruppe, umfassend horizontale Stirnwölbung, vertikale Stirnwölbung, Stirnbreite, frontonasaler Winkel, frontooccipitaler Durchmesser, biparietaler Durchmesser, horizontaler Krümmungsradius des Occipitalmoduls, auf einer Perzentilenkurve liegt, die 90% < x% ≤ 97% ist, bezogen auf eine normal verteilte Population von Kindern im Alter von 0-1 Jahren, wobei bevorzugt ist, daß es auf einer Perzentilenkurve liegt, die 97% ist, bezogen auf eine normal verteilte Population von Kindern im Alter von 0-1 Jahren.

In einer Ausführungsform ist vorgesehen, daß das erfindungsgemäße dreidimensionale Modell auf der jeweiligen Perzentilenkurve im Lebensalterbereich von Kindern im Alter von 12-20 Wochen liegt.

In einer anderen Ausführungsform ist vorgesehen, daß das erfindungsgemäße dreidimensionale Modell auf der jeweiligen Perzentilenkurve im Lebensalterbereich von Kindern im Alter von 24-32 Wochen liegt.

Es ist bevorzugt, daß das erfindungsgemäße dreidimensionale Modell in seinen äußeren Dimensionen der Knochenstärke eines menschlichen Kinderschädels entsprechend um 1-5 mm reduziert ist. Der entsprechende Wert kann patientenabhängig innerhalb des angegebenen Bereichs variieren.

In einer Ausführungsform ist vorgesehen, daß das erfindungsgemäße dreidimensionale Modell eine Formschale umfaßt, die, abnehmbar, einem inneren Stützelement aufliegt, wobei bevorzugt ist, daß die Formschale den Stirnteil (Frontalmodul), den Scheitelteil (Parietalmodul) und den Hinterhauptteil (Occipitalmodul), die, besonders bevorzugt, untereinander verbunden sind, umfaßt.

In einer Ausführungsform ist vorgesehen, daß das innere Stützelement ein Gestell oder Rahmen ist, auf dem die Formschale aufliegt.

In einer Ausführungsform ist vorgesehen, daß die äußere Oberfläche der Formschale Aussparungen zur Aufnahme von Stabilisierungselementen enthält, wobei bevorzugt ist, daß die Aussparungen Öffnungen in der Formschale sind, bevorzugt streifen- und/oder punktförmige Öffnungen.

In einer Ausführungsform ist vorgesehen, daß die Aussparungen auf der äußeren Oberfläche der Formschale in regelmäßigen Abständen zueinander angeordnet sind.

In einer Ausführungsform ist vorgesehen, daß die Formschale Aussparungen aufweist, die eine Verbindung von auf die äußere Oberfläche der Formschale und/oder in die Aussparungen aufgebrachten Stabilisierungselementen mit ebenfalls von außen auf die Formschale aufgebrachten Knochenplatten ermöglichen, wobei bevorzugt ist, daß die Verbindung mittels Verschraubung oder Vernietung stattfindet, und wobei besonders bevorzugt ist, daß die zuletztgenannten Aussparungen ganz oder teilweise mit den davorgenannten Aussparungen identisch sind.

Die Aufgaben der Erfindung werden auch gelöst durch ein dreidimensionales Modell gemäß der vorliegenden Erfindung zur Verwendung in einem Verfahren zur operativen Behandlung einer Craniosynostose.

Weiterhin werden die Aufgaben der Erfindung gelöst durch einen Kit, umfassend mindestens ein, bevorzugt mindestens zwei, bevorzugt mindestens drei unterschiedliche Modelle gemäß der vorliegenden Erfindung, wobei bevorzugt ist, daß das Kit fünf, bevorzugt sechs unterschiedliche Modelle gemäß der vorliegenden Erfindung umfaßt.

In einer Ausführungsform ist vorgesehen, daß der Kit weiterhin ein oder mehrere Teile umfaßt, ausgewählt aus der Gruppe:
- resorbierbare Stabilisierungselemente, insbesondere Platten oder Streifen, die zum Stabilisieren von Schädelknochenfragmenten geeignet sind,
- resorbierbare Schrauben und/oder Nieten zum Fixieren von Schädelknochenfragmenten auf den Stabilisierungselementen,
- Werkzeug zum Einbringen und/oder Entfernen der Schrauben oder Nieten, insbesondere Schraubendreher und Schraubenentferner.

Dabei ist bevorzugt, daß die resorbierbaren Stabilisierungselemente in erhitztem Zustand formbar sind, wobei besonders bevorzugt ist, daß die resorbierbaren Stabilisierungselemente resorbierbare Milchsäureplatten oder -streifen sind.

Solche resorbierbaren Milchsäureplatten oder -streifen sind kommerziell erhältlich.

In einer Ausführungsform ist vorgesehen, daß die Platten oder Streifen physiologisch akzeptable Dicke, Breite und Länge aufweisen, wobei ein Kit einheitlich große Platten und Streifen oder unterschiedlich große Platten und Streifen enthält.

Die Dimensionen der Platten und Streifen sind bevorzugt patientenabhängig auszuwählen. Dabei ist vorgesehen, daß der Kit bevorzugt unterschiedlich große Platten und/oder Streifen enthält, um den jeweiligen individuellen Gegebenheiten des jeweiligen Operationssitus Rechnung tragen zu können.

Die Aufgaben der Erfindung werden auch durch einen erfindungsgemäßen Kit zur Verwendung in einem Verfahren zur operativen Behandlung einer Craniosynostose gelöst.

Weiterhin werden die Aufgaben der Erfindung gelöst durch ein Verfahren zur operativen Behandlung einer Craniosynostose bei einem Patienten, umfassend die Schritte:
- Steriles Bereitstellen eines dem Patienten nach Vergleichmessung ähnlichsten dreidimensionalen Modells gemäß der vorliegenden Erfindung,
- Auflegen von in erhitztem Zustand formbaren Stabilisierungselementen, bevorzugt resorbierbaren Milchsäureplatten, auf das Modell und Aushärtung der Stabilisierungselemente,
- Entnahme eines von der Craniosynostose betroffenen, fehlgebildeten Kalottenabschnitts und Auftrennung des Kalottenabschnitts in Fragmente, bevorzugt Streifen,
- Aufformung der Fragmente des fehlgebildeten Kalottenabschnitts auf das dreidimensionale Modell durch Ausdünnung, Destabilisierung und/oder Modellierung,
- Fixierung der Fragmente auf den darunterliegenden Stabilisierungselementen, bevorzugt durch Verschraubung und/oder Vernietung, wodurch ein Implantat aus Fragmenten und Stabilisierungselementen gebildet wird,
- Implantation des Implantats durch Einpassung und Fixierung, bevorzugt Verschraubung, in dem Schädel des Patienten.

Es ist bevorzugt, daß das dreidimensionale Modell der Knochenstärke entsprechend um 1-5 mm in seinen äußeren Dimensionen reduziert ist, um eine formgerechte Implantation der umgeformten Knochenfragmente zu ermöglichen.

In einer Ausführungsform ist vorgesehen, daß zur Aufformung der Fragmente des fehlgebildeten Kalottenabschnitts auf das dreidimensionale Modell diese auf das Modell aufgebracht und unter Krafteinwirkung, bevorzugt mittels eines reversibel entfernbaren Fixiermittels, bevorzugt mittels eines elastischen Bands, in Position gehalten werden.

Es ist bevorzugt, daß zur Implantation das Implantat von dem dreidimensionalen Modell abgenommen wird.

Wesentlicher Ansatzpunkt der Erfindung ist die Umsetzung eines neuen Verfahrens der statistischen Modellbildung, das erstmals valide Daten zur Normalverteilung kindlicher Kopfformen bietet. Dabei haben die Erfinder überraschenderweise gefunden, daß durch den Einsatz eines geeigneten, individuell an den jeweiligen Patienten angepaßten dreidimensionalen Modells, welches sterilisierbar ist, gute Erfolge in planbarer und rational nachzuvollziehender Weise erzielt werden können.

Vorbereitend werden innerhalb einer bezüglich des Schädelwachstums gesunden Gruppe von Rekonstruktionen der Altersstufen 12 -20 Wochen (4 Monate) und 24-32 Wochen (8 Monate) typische Formmerkmale (Horizontale Stirnwölbung, Vertikale Stirnwölbung, Stirnbreite, Frontonasaler Winkel, frontooccipitaler Durchmesser, biparietaler Durchmesser, horizontaler Krümmungsradius der Occipitalschuppe etc.) erfaßt und analysiert. Grundsätzlich alle Meßwerte werden in Perzentilenkurven dokumentiert. Eine Perzentilenkurve, wie hierin verwendet, dient zum Vergleich eines jeweiligen Patienten mit anderen gleichaltrigen Patienten. Dabei bedeutet eine 10% Perzentile, daß 9% der gleichaltrigen Patienten hinsichtlich eines bestimmten Merkmals unter dem Wert des untersuchten Patienten, 90% der gleichaltrigen Patienten hinsichtlich desselben Merkmals über dem Wert des untersuchten Patienten liegen. Aus der Fusion der Rekonstruktionen altersidentischer Kinder wird ein virtueller Durchschnittskopf "4 Monate" bzw. "8 Monate" (∼ 50% Perzentile) sowie je ein Kopf mit maximaler Formabweichungen einzelner Merkmale (Kopflänge, -breite, Stirnbreite, -wölbung, etc.) (∼ 3% Perzentile bzw. ∼ 97% Perzentile) berechnet und in stereolithographische Modelle umgesetzt.

Dabei soll möglichst die gesamte Variationsbreite unterschiedlicher Merkmale erfaßt werden. Pro Altersgruppe sind für jeden Kalottenabschnitt etwa 6 variierende Teilmodelle vorgesehen, die dann je nach gegebener Kopfform des Patienten und gewünschter Veränderung ausgesucht werden können.

In der Umsetzung der Daten kann im Gegensatz zu bisher üblichen bildschirmgebunden Operationsplanungsverfahren sowohl die Erzeugung von Kosten (und Risiken) durch individuelle Bildgebung vermieden werden als auch die Präzision der Umsetzung der Formvorgabe ohne Einsatz etwa von bildabhängigen Navigationssystemen ermöglicht werden.

Dazu wird aus dem Datenpool ein Instrumentarium, basierend auf einem altersbezogenen Sortiment dreidimensionaler mehrteiliger Formmodule entwickelt. Fehlgeformte Kalottenabschnitte können dann über einem passenden erfindungsgemäßen Modell präzise umgeformt mit vorgeformten resorbierberen Stabilisierungselementen innenseitig versteift und "en bloc" reimplantiert werden).

Der Einsatz eines erfindungsgemäßen Modells oder mehrerer erfindungsgemäßer Modelle ermöglicht den Ersatz der suboptimalen Transposition von Fragmenten durch die tatsächliche mehrdimensionale Umformung unter Korrektur der Krümmungsradien auf einer formgebenden Oberfläche.

Besonderer Vorteil dieses Verfahrens ist darüber hinaus die kosmetisch günstigere innenseitige Lage der relativ dicken resorbierbaren Stabilisierungselemente.

Mit dem hier vorgestellten erfindungsgemäßen Operationskonzept bzw. den erfindungsgemäßen dreidimensionalen Modellen bzw. Kit wird dieses Ergebnis planbar und im Vergleich mit dem Ausgangsmodell der Operation jederzeit kontrollierbar. Korrekturverluste durch pathologisches Knochenwachstum können quantifiziert werden und langfristig in die Operationsvorgaben (Modellplanung) einfließen. Die Anzahl suboptimaler Ergebnisse und die Häufigkeit von Zweiteingriffen bei nichtsyndromalen Synostosen reduziert sich entsprechend.

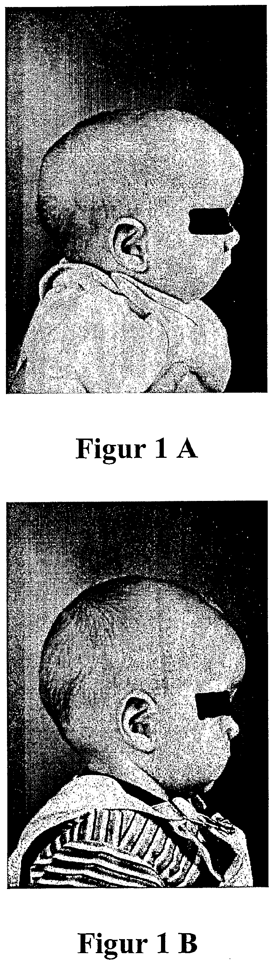

Es wird nunmehr Bezug genommen auf die Figuren, bei denen
Abbildung 1 das Beispiel eines vier Monate alten Säuglings mit Sagittalnahtsynostose vor (Fig. 1 A), und vier Wochen nach (Fig. 1 B) einer operativer Umformung zeigt,

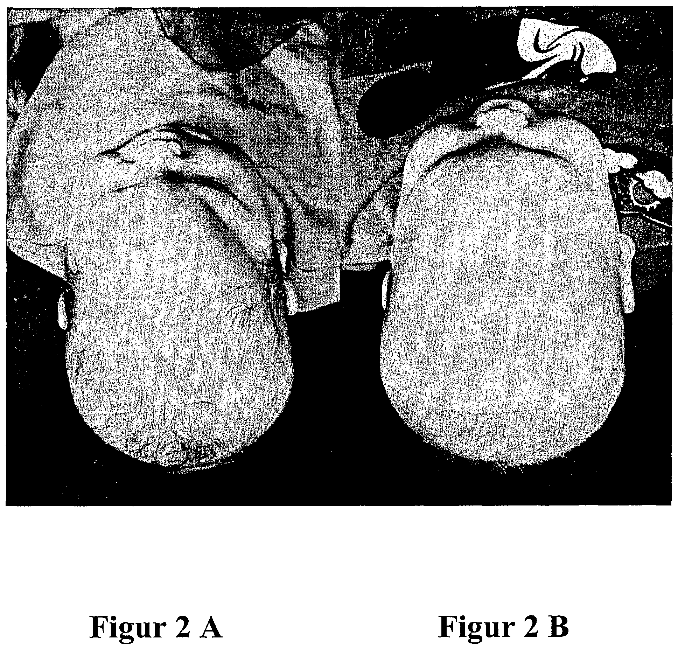

Abbildung 2 das Beispiel eines fünf Monate alten Säuglings mit Frontalnahtsynostose vor (Fig. 2 A), und vier Wochen nach (Fig. 2 B) einer operativer Umformung zeigt,

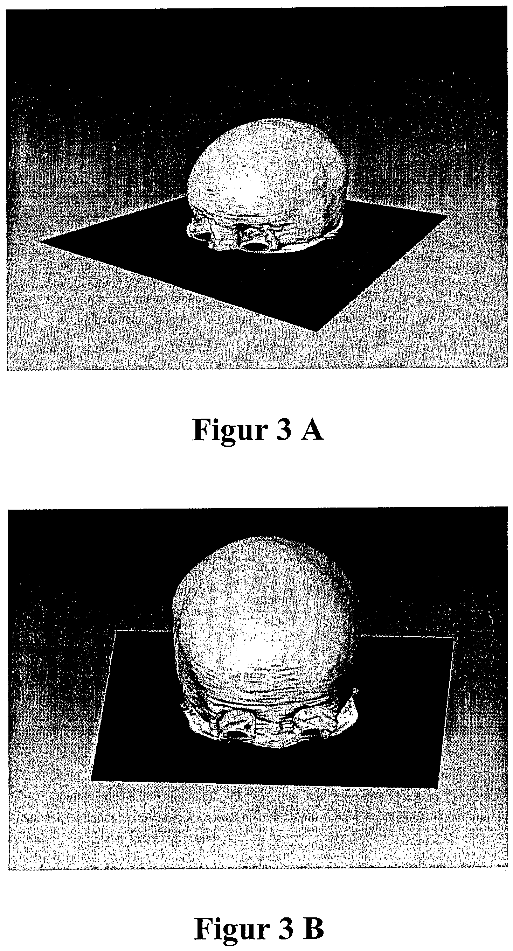

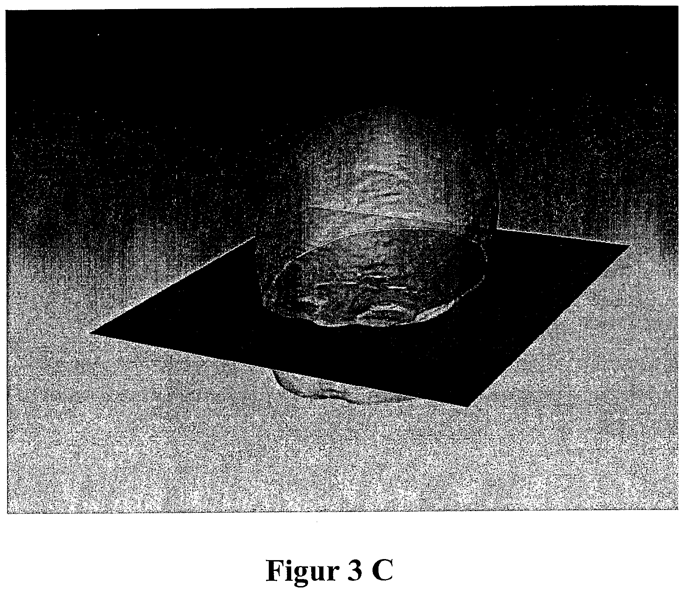

Abbildung 3 die manuelle Segmentierung einer kindlichen Schädelkalotte aus einem MRT-Datensatz (Magnetresonanztomographie) zeigt (Fig. 3 A - 3 C),

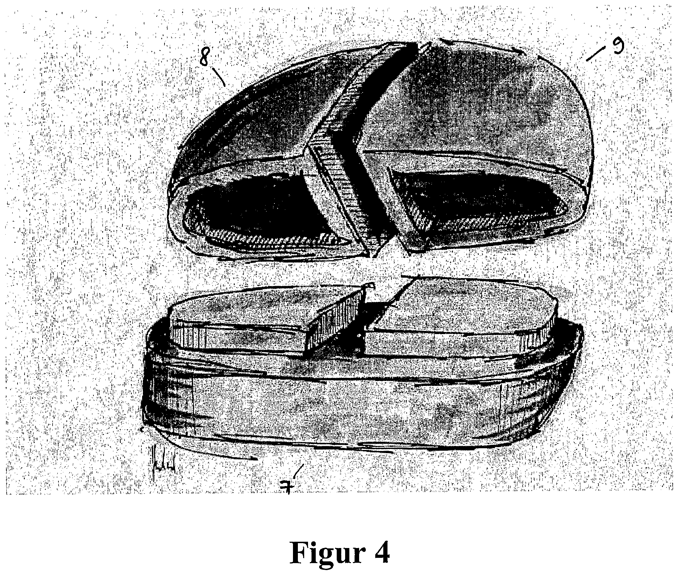

Abbildung 4 ein Beispiel eines sterilisierbaren Modells einer Schädelkalotte, bestehend aus Basis- 7, Frontal- 8 und Parieto-Occipitalmodul 9 zeigt,
Abbildung 5 die Aufformung eines wärmebiegsamen resorbierbaren Stabilisierungselementes 4 auf ein Stirnmodul 8 zeigt,
Abbildung 6 eine Aufformung des destabilisierten Knochenstreifen 10 auf das ausgehärtete Stabilisierungselement 4 über einem Stirnmodul zeigt,
Abbildung 7 ein fertig verschraubtes Implantat (Knochenstreifen auf Stabilisierungselementen) auf einem Stirnmodul zeigt,
Abbildung 8 die Reimplantation des umgeformten Stirnsegmentes in den vorbereiteten Situs zeigt,
Abbildung 9 eine auf einem inneren Stützelement 2 abnehmbar aufliegende Formschale 1 zeigt, und
Abbildung 10 die auf der Formschale aufgeformten Knochenfragmente 10 mit Stabilisierungselementen 4 zeigt, die von einem Fixierungsmittel 11, hier einem elastischen Band, in Position gehalten werden.

### Vorarbeiten zur statistischen Modellbildung

Die Erstellung von 3D Atlanten und deren Nutzung zur 3D Formanalyse ist gegenwärtig Gegenstand der Forschung in diversen Bereichen, wie z.B. der Anthropometrie, der medizinischen Bildanalyse, Ergonomiestudien. Ein 3D Atlas beschreibt dabei die mittlere Form eines Objektes und seine typischen Abweichungen innerhalb einer gegebenen Stichprobe. Die Erzeugung eines Atlas erfordert einen Formvergleich, wobei man zwischen flächen- und volumenbasierten Ansätzen unterscheidet. Das Ergebnis des Formvergleiches ermöglicht die Darstellbarkeit der Stichprobe in einem hochdimensionalen Vektorraum. In der Literatur existieren eine Reihe von Verfahren zur Extraktion relevanter statistischer Parameter, die zur Erstellung eines speziellen Atlas verwendet werden.

Zur Erstellung der Oberflächenmodelle für den 3D Atlas ist die Segmentierung (Gewebeklassifikation) von tomografischen Bilddaten erforderlich. Hierzu gibt es Softwaresysteme, die den Segmentierungsvorgang mittels manueller und semiautomatischer Werkzeuge unterstützen (Analyze, Amira, Materialise).

Ein vielversprechender Ansatz zur vollautomatischen Segmentierung basiert auf der Nutzung von ä priori Wissen in Form von 3D Atlanten. Der Atlas wird zunächst auf Basis der segmentierten Daten erzeugt. Sobald er sich aber aus einer hinreichend großen Zahl an Modellen zusammensetzt, kann er auch selbst für die Segmentierung eingesetzt werden und somit den Segmentierungsvorgang vereinfachen und beschleunigen. Die ständige Erweiterung eines Atlas ist erforderlich, um mit einer bestimmten Wahrscheinlichkeit Aussagen über die generelle Form eines Objektes treffen zu können.

### Beschreibung einer bevorzugten Ausführungsform des erfindungsgemäßen Operationsverfahrens unter Verwendung von erfindungsgemäßen Modellen

Nach abgeschlossener statistischer Modellerzeugung werden für eine spezifische Altersgruppe (z.B. 4 Monate) 5-8 repräsentative Formvarianten produziert. Das dem individuellen Patienten nach Vergleichsmessung ähnlichste Modell wird steril im Operationssaal bereitgestellt.

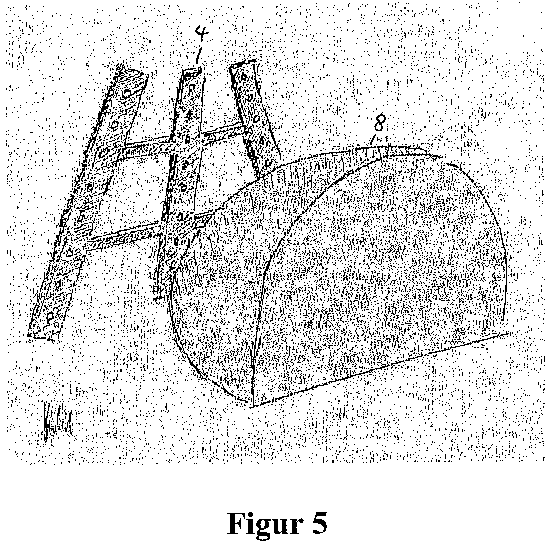

Auf das bereits um Kalottendurchmesser und Plattendurchmesser reduzierte Modell werden nun in erhitztem Zustand formbare, bevorzugt resorbierbare Stabilisierungselemente 4 (bspw. resorbierbare Milchsäureplatten) aufgelegt (Fig. 5).

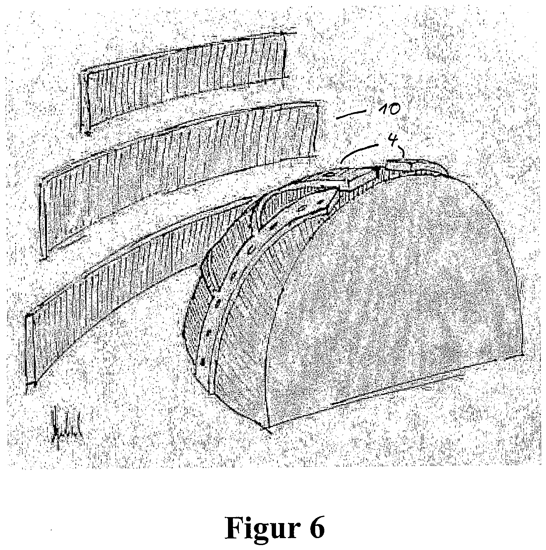

Nach Aushärtung der Formelemente wird der in Knochenstreifen zertrennte fehlgebildete Kalottenabschnitt 10 aufgeformt. Dazu werden die einzelnen Fragmente 10 ausgedünnt, destabilisiert und modelliert (Fig. 6).

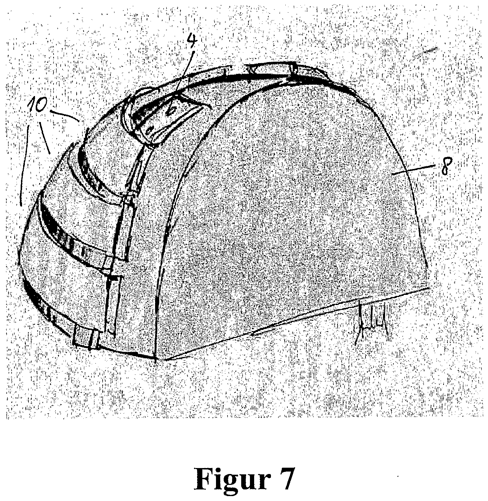

Die anliegenden Knochenstreifen 10 werden nun mit der darunterliegenden Platte aus resorbierbaren Stabilisierungselementen 4 verschraubt (Fig. 7).

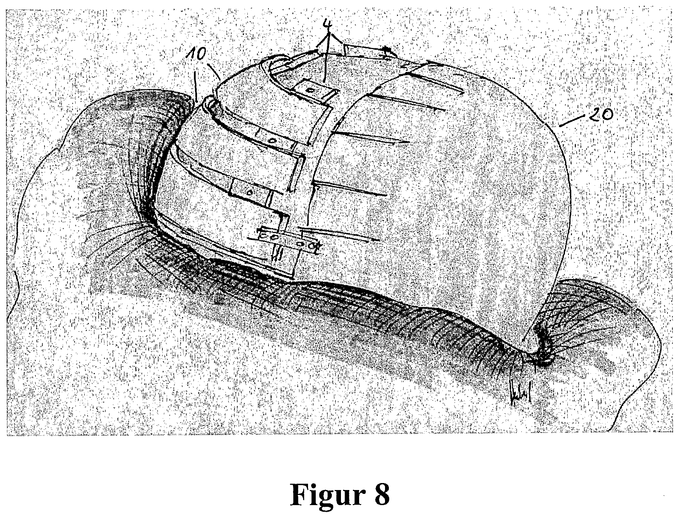

Anschließend wird der fertig umgeformte und stabilisierte Kalottenabschnitt 10 in den Situs eingebracht, durch kurze Erwärmung der Platten 4 feinangepaßt und mit der verbliebenen Kalotte 20 verschraubt (Fig. 8).

### Beschreibung einer besonders bevorzugten Ausführungsform eines erfindungsgemäßen dreidimensionales Modells

### Formschale

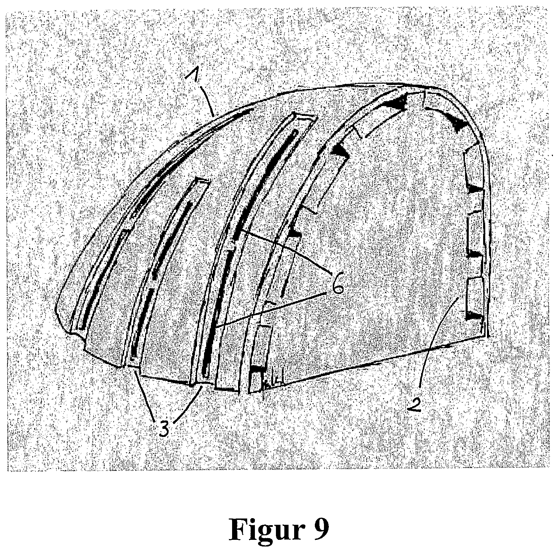

Eine bevorzugte Ausführungsform, wie in Fig. 9 gezeigt, des erfindungsgemäßen dreidimensionalen Modells umfaßt mindestens eine Formschale 1, die einem inneren Stützelement 2 abnehmbar aufliegt. Die äußeren Abmessungen der Formschale sind der Knochenstärke entsprechend, um 1-5 mm, bevorzugt um 3 mm reduziert. In die Oberfläche sind regelmäßige Einlassungen oder Aussparungen 3 zur Aufnahme verschiedener Stabilisierungselemente 4 eingearbeitet. Die Aussparungen 3 weisen schlitzförmige Öffnungen 6 auf, die einen Zugriff auf in den Aussparungen 3 eingelegten Stabilisierungselementen vom Inneren der Schale ermöglichen. Die Formschale umfaßt einen Stirnteil, einen Scheitelteil und einen Hinterhauptteil, die einzeln getrennt oder miteinander verbunden vorliegen können. In einer besonders bevorzugten Ausführungsform sind Scheitelteil und Hinterhauptteil miteinander verbunden und ergeben einen Scheitel- und Hinterhauptteil (Parieto-Occipitalmodul).

### Rumpf

Die Formschale 1 liegt - abnehmbar - einem inneren Stützelement 2 auf. Das Stützelement ist in einer bevorzugten Ausführungsform ein rahmenartiges Gerüst.

### Stabilisierungselemente

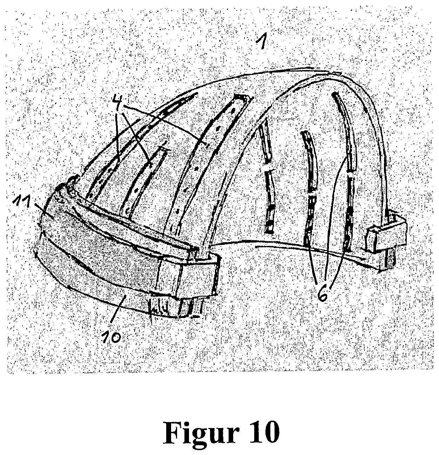

Die Einlassungen oder Aussparungen 3 der Schale werden nun, wie auch in Fig. 10 gezeigt, nach Wunsch mit erhitzt formbaren Stabilisierungselementen 4 ausgekleidet. Abweichend von der bisherigen traditionellen Operationstechnik wird nun der fehlgebildete Knochen in regelmäßige Streifen geteilt und destabilisiert. Die einzelnen Streifen werden aufgeformt und mit einem elastischen Band 5 in Position gehalten.

Anschließend wird die Schale abgenommen und durch die schlitzförmigen inneren Öffnungen 6 eine stabile Verschraubung oder Vernietung der Stabilisierungselemente mit den Knochenstreifen 10 vorgenommen.

Das fertige Implantat wird dann von der Schale genommen, fein angepaßt und en bloc implantiert.

Die in der vorstehenden Beschreibung, den Ansprüchen sowie den Zeichnungen offenbarten Merkmale der Erfindung können sowohl einzeln als auch in beliebigen Kombinationen für die Verwirklichung der Erfindung in ihren verschiedenen Ausführungsformen wesentlich sein

## Patentansprüche

1. Dreidimensionales sterilisierbares Modell eines menschlichen Schädels in Lebensgröße, umfassend einen Scheitelteil und einen Hinterhauptteil, **dadurch gekennzeichnet, daß** es einen Stirnteil (8) und zusätzlich einen Basisteil (7) umfaßt, auf dem Stirnteil (8), Scheitelteil und Hinterhauptteil abnehmbar angeordnet sind, weiterhin **dadurch gekennzeichnet, dass** es das Modell eines Kinderschädels ist.

2. Dreidimensionales Modell nach Anspruch 1, **dadurch gekennzeichnet, daß** mindestens zwei Teile, ausgewählt aus Stirnteil (8), Scheitelteil und Hinterhauptteil, miteinander verbunden sind.

3. Dreidimensionales Modell nach einem der Ansprüche 1-2, **dadurch gekennzeichnet, daß** der Scheitelteil und Hinterhauptteil miteinander zu einem Scheitel- und Hinterhauptteil (9) verbunden sind.

4. Dreidimensionales Modell nach einem der vorangehenden Ansprüche, **dadurch gekennzeichnet, daß** es einen durchschnittlichen Kinderschädel darstellt, wobei der Durchschnitt auf eine normal verteilte Population von Kindern bezogen ist, die 12-20 Wochen alt sind.

5. Dreidimensionales Modell nach einem der vorangehenden Ansprüche, **dadurch gekennzeichnet, daß** es einen durchschnittlichen Kinderschädel darstellt, wobei der Durchschnitt auf eine normal verteilte Population von Kindern bezogen ist, die 24-32 Wochen alt sind.

6. Dreidimensionales Modell nach einem der Ansprüche4-5, **dadurch gekennzeichnet, daß** es hinsichtlich eines oder mehrerer der Merkmale, ausgewählt aus der Gruppe, umfassend horizontale Stirnwölbung, vertikale Stirnwölbung, Stirnbreite, frontonasaler Winkel, frontooccipitaler Durchmesser, biparietaler Durchmesser und horizontaler Krümmungsradius des Occipitalmoduls, einen durchschnittlichen Kinderschädel darstellt.

7. Dreidimensionales Modell nach einem der Ansprüche 1-3, **dadurch gekennzeichnet, daß** es hinsichtlich eines oder mehrerer der Merkmale, ausgewählt aus der Gruppe, umfassend horizontale Stirnwölbung, vertikale Stirnwölbung, Stirnbreite, frontonasaler Winkel, frontooccipitaler Durchmesser, biparietaler Durchmesser und horizontaler Krümmungsradius des Occipitalmoduls, auf einer Perzentilenkurve liegt, die ≤ 10% ist, bezogen auf eine normal verteilte Population von Kindern im Alter von 0-1 Jahren.

8. Dreidimensionales Modell nach Anspruch7, **dadurch gekennzeichnet, daß** es auf einer perzentilen Kurve liegt, die 3% ≤ x% ≤ 10% ist, bevorzugt 3% ist, bezogen auf eine normal verteilten Population von Kindern im Alter von 0-1 Jahren.

9. Dreidimensionales Modell nach einem der Ansprüche 1-3, **dadurch gekennzeichnet, daß** es hinsichtlich eines oder mehrerer der Merkmale, ausgewählt aus der Gruppe, umfassend horizontale Stirnwölbung, vertikale Stirnwölbung, Stirnbreite, frontonasaler Winkel, frontooccipitaler Durchmesser, biparietaler Durchmesser, horizontaler Krümmungsradius des Occipitalmoduls, auf einer Perzentilenkurve liegt, die 10% < x% ≤ 30% ist, bezogen auf eine normal verteilte Population von Kindern im Alter von 0-1 Jahren.

10. Dreidimensionales Modell nach einem der Ansprüche 1-3, **dadurch gekennzeichnet, daß** es hinsichtlich eines oder mehrerer der Merkmale, ausgewählt aus der Gruppe, umfassend horizontale Stirnwölbung, vertikale Stirnwölbung, Stirnbreite, frontonasaler Winkel, frontooccipitaler Durchmesser, biparietaler Durchmesser, horizontaler Krümmungsradius des Occipitalmoduls, auf einer Perzentilenkurve liegt, die 30% < x% ≤ 70% ist, bezogen auf eine normal verteilte Population von Kindern im Alter von 0-1 Jahren.

11. Dreidimensionales Modell nach Anspruch 10, **dadurch gekennzeichnet, daß** es auf einer Perzentilenkurve liegt, die 45% ≤ x% ≤ 55% ist, bezogen auf eine normal verteilte Population von Kindern im Alter von 0-1 Jahren.

12. Dreidimensionales Modell nach einem der Ansprüche 10-11, **dadurch gekennzeichnet, daß** es auf einer Perzentilenkurve liegt, die 50% ist, bezogen auf eine normal verteilte Population von Kindern im Alter von 0-1 Jahren.

13. Dreidimensionales Modell nach einem der Ansprüche 1-3, **dadurch gekennzeichnet, daß** es hinsichtlich eines oder mehrerer der Merkmale, ausgewählt aus der Gruppe, umfassend horizontale Stirnwölbung, vertikale Stirnwölbung, Stirnbreite, frontonasaler Winkel, frontooccipitaler Durchmesser, biparietaler Durchmesser, horizontaler Krümmungsradius des Occipitalmoduls, auf einer Perzentilenkurve liegt, die 55% < x% ≤ 70% ist, bezogen auf eine normal verteilte Population von Kindern im Alter von 0-1 Jahren.

14. Dreidimensionales Modell nach einem der Ansprüche 1-3, **dadurch gekennzeichnet, daß** es hinsichtlich eines oder mehrerer der Merkmale, ausgewählt aus der Gruppe, umfassend horizontale Stirnwölbung, vertikale Stirnwölbung, Stirnbreite, frontonasaler Winkel, frontooccipitaler Durchmesser, biparietaler Durchmesser, horizontaler Krümmungsradius des Occipitalmoduls, auf einer Perzentilenkurve liegt, die 70% < x% ≤ 90% ist, bezogen auf eine normal verteilte Population von Kindern im Alter von 0-1 Jahren.

15. Dreidimensionales Modell nach einem der Ansprüche 1-3, **dadurch gekennzeichnet, daß** es hinsichtlich eines oder mehrerer der Merkmale, ausgewählt aus der Gruppe, umfassend horizontale Stirnwölbung, vertikale Stirnwölbung, Stirnbreite, frontonasaler Winkel, frontooccipitaler Durchmesser, biparietaler Durchmesser, horizontaler Krümmungsradius des Occipitalmoduls, auf einer Perzentilenkurve liegt, die 90% < x% ≤ 97% ist, bezogen auf eine normal verteilte Population von Kindern im Alter von 0-1 Jahren.

16. Dreidimensionales Modell nach Anspruch15, **dadurch gekennzeichnet, daß** es auf einer Perzentilenkurve liegt, die 97% ist, bezogen auf eine normal verteilte Population von Kindern im Alter von 0-1 Jahren.

17. Dreidimensionales Modell eines menschlichen Kinderschädels in Lebensgröße nach einem der Ansprüche 7-16, **dadurch gekennzeichnet, daß** es auf der jeweiligen Perzentilenkurve im Lebensalterbereich von Kindern im Alter von 12-20 Wochen liegt.

18. Dreidimensionales Modell eines menschlichen Kinderschädels in Lebensgröße nach einem der Ansprüche 7-16, **dadurch gekennzeichnet, daß** es auf der jeweiligen Perzentilenkurve im Lebensalterbereich von Kindern im Alter von 24-32 Wochen liegt.

19. Dreidimensionales Modell nach einem der Ansprüche 1-18, **dadurch gekennzeichnet, daß** es in seinen äußeren Dimensionen der Knochenstärke eines menschlichen Kinderschädels entsprechend um 1-5 mm reduziert ist.

20. Dreidimensionales Modell nach einem der Ansprüche 1-19 **dadurch gekennzeichnet, daß** es eine Formschale (1) umfaßt, die, abnehmbar, einem inneren Stützelement (2) aufliegt.

21. Dreidimensionales Modell nach Anspruch 20, **dadurch gekennzeichnet, daß** die Formschale (1) den Stirnteil (8), den Scheitelteil und den Hinterhauptteil, die untereinander verbunden sind, umfaßt.

22. Dreidimensionales Modell nach einem der Ansprüche 20-21, **dadurch gekennzeichnet, daß** das innere Stützelement (2) ein Gestell oder Rahmen ist, auf dem die Formschale (1) aufliegt.

23. Dreidimensionales Modell nach einem der Ansprüche 20-22, **dadurch gekennzeichnet, daß** die äußere Oberfläche der Formschale Aussparungen (3) zur Aufnahme von Stabilisierungselementen (4) enthält.

24. Dreidimensionales Modell nach Anspruch 23, **dadurch gekennzeichnet, daß** die Aussparungen (3) Öffnungen in der Formschale sind, bevorzugt streifen- und/oder punktförmige Öffnungen.

25. Dreidimensionales Modell nach einem der Ansprüche 23-24, **dadurch gekennzeichnet, daß** die Aussparungen (3) auf der äußeren Oberfläche der Formschale in regelmäßigen Abständen zueinander angeordnet sind.

26. Dreidimensionales Modell nach einem der Ansprüche 20-24, **dadurch gekennzeichnet, daß** die Formschale Aussparungen (3) aufweist, die eine Verbindung von auf die äußere Oberfläche der Formschale und/oder in die Aussparungen (3) aufgebrachten Stabilisierungselementen mit ebenfalls von außen auf die Formschale aufgebrachten Knochenplatten (10) ermöglichen, wobei bevorzugt ist, daß die Verbindung mittels Verschraubung oder Vernietung stattfindet.

27. Dreidimensionales Modell nach Anspruch 26, **dadurch gekennzeichnet, daß** die Aussparungen (3) nach Anspruch 26 ganz oder teilweise mit den Aussparungen (3) nach einem der Ansprüche 23-25 identisch sind.

28. Kit, umfassend mindestens ein, bevorzugt mindestens zwei, bevorzugt mindestens drei unterschiedliche Modelle nach einem der Ansprüche 1-27.

29. Kit nach Anspruch 28, umfassend fünf, bevorzugt sechs unterschiedliche Modelle nach einem der Ansprüche 1-27.

30. Kit nach einem der Ansprüche 28-29, weiterhin umfassend ein oder mehrere Teile ausgewählt aus der Gruppe:
- resorbierbare Stabilisierungselemente (4), insbesondere Platten oder Streifen, die zum Stabilisieren von Schädelknochenfragmenten geeignet sind,
- resorbierbare Schrauben und/oder Nieten zum Fixieren von Schädelknochenfragmenten auf den Stabilisierungselementen,
- Werkzeug zum Einbringen und/oder Entfernen der Schrauben oder Nieten, insbesondere Schraubendreher und Schraubenentferner.

31. Kit nach Anspruch 30, **dadurch gekennzeichnet, daß** die resorbierbaren Stabilisierungselemente (4) in erhitztem Zustand formbar sind.

32. Kit nach Anspruch 31, **dadurch gekennzeichnet, daß** die resorbierbaren Stabilisierungselemente (4) resorbierbare Milchsäureplatten oder -streifen sind.

33. Kit nach einem der Ansprüche 30-32, **dadurch gekennzeichnet, daß** die Platten oder Streifen physiologisch akzeptable Dicke, Breite und Länge aufweisen, wobei ein Kit einheitlich große Platten und Streifen oder unterschiedlich große Platten und Streifen enthält.

34. Verfahren zur Planung und Vorbereitung einer Operation eines Patienten mit Cranialsynostose, umfassend die Schritte:
- Erfassung und Analyse von Schädelformmerkmalen in einer Gruppe von bezüglich des Schädelwachstums gesunden Patienten,
- Berechnung eines virtuellen Kinderschädels, basierend auf den erfassten und analysierten Schädelformmerkmalen,
- Umsetzung des berechneten Kinderschädels in ein stereolithographisches Modell nach einem der Ansprüche 1-27.

35. Verfahren nach Anspruch 34, **dadurch gekennzeichnet, daß** der Kinderschädel ein durchschnittlicher Kinderschädel ist.

36. Verfahren nach Anspruch 35, **dadurch gekennzeichnet, daß** neben dem durchschnittlichen Kinderschädel ein Kinderschädel mit maximaler Formabweichung einzelner Merkmale, wie Kopflänge, -breite, Stirnbreite, oder -wölbung, berechnet und/oder umgesetzt wird.

37. Verfahren nach einem der Ansprüche 34-36, **dadurch gekennzeichnet, daß** die Schädelformmerkmale ausgewählt sind aus horizontaler Stirnwölbung, vertikaler Stirnwölbung, Stirnbreite, frontonasaler Winkel, frontooccipitaler Durchmesser, biparietaler Durchmesser, horizontaler Krümmungsradius der Occipitalschuppe, Kopflänge, Kopfbreite, Stirnwölbung.

38. Verfahren nach einem der Ansprüche 34-37, **dadurch gekennzeichnet, daß** die Erfassung und Analyse von Schädelformmerkmalen in einer bezüglich des Schädelwachstums gesunden Gruppe von Patienten der Alterstufen 12-20 Wochen und 24-32 Wochen stattfindet.

39. Verfahren nach einem der Ansprüche 34-38, **dadurch gekennzeichnet, daß** alle Messwerte in perzentilen Kurven dokumentiert werden.

40. Verfahren nach einem der Ansprüche 34-39, **dadurch gekennzeichnet, daß** ein virtueller Durchschnittschädel für Kinder im Alter von 4 bzw. 8 Monaten sowie je ein Schädel mit maximaler Formabweichung einzelner Schädelformmerkmale berechnet wird.

41. Verfahren nach einem der Ansprüche 36-40, **dadurch gekennzeichnet, daß** der Durchschnittschädel und der/die Schädel mit maximaler Formabweichung in 5-8 stereolithografische Modelle nach einem der Ansprüche 1-27 umgesetzt werden.

42. Verfahren nach einem der Ansprüche 34-41 umfassend die Schritte:
- Bereitstellen von Schädelrekonstruktionen von bezüglich des Schädelwachstums gesunden Patienten der Alterstufen 12-20 Wochen und 24-32 Wochen,
- Erfassung und Analyse von typischen Schädelformmerkmalen, wie etwa horizontale Stirnwölbung, vertikale Stirnwölbung, Stirnbreite, frontonasaler Winkel, frontooccipitaler Druchmesser, biparietaler Durchmesser, horizontaler Krümmungsradius der Occipitalschuppe,
- Dokumentation aller Messwerte in Perzentilen
- Berechnung eines virtuellen Durchschnittsschädels für Kinder im Alter von 4 bzw. 8 Monaten und/oder je eines Schädels mit maximaler Formabweichung einzelner Merkmale, wie Kopflänge, Kopfbreite, Stirnbreite oder Stirnwölbung,
- Umsetzung des Durchschnittsschädels und/oder des Schädels/der Schädel mit maximaler Formabweichug in 5-8 stereolithographische sterilisierbare Modelle nach einem der Ansprüche 1-27.

43. Verfahren nach Anspruch 42, weiterhin umfassend die Schritte:
- Auswahl des nach Vergleichsmessung dem Patienten ähnlichsten stereolithographischen Modells,
- sterile Bereitstellung des Modells im Operationssaal.

## Claims

1. A three-dimensional sterilisable model of a human skull in life size, comprising an parietal part and a posterior part, **characterized in that** it further comprises a frontal part (8) and additionally a base part (7) onto which the frontal part (8), the parietal part and the occipital part are detachably arranged, characterized further **in that** it is the model of a child's skull.

2. Three-dimensional model according to claim 1, **characterized in that** at least two parts, selected from the frontal part (8), the parietal part and the occipital part, are connected to one another.

3. Three-dimensional model according to one of claims 1 - 2, **characterized in that** the parietal part and the occipital part are connected to one another to form a parietal occipital part (9).

4. Three-dimensional model according to one of the preceding claims, **characterized in that** it represents an average child's skull, the average being related to a normally distributed population of children who are 12-20 weeks old.

5. Three-dimensional model according to one of the preceding claims, **characterized in that** it represents an average child's skull, the average of a normal distribution population of children aged 24-32 weeks.

6. Three-dimensional model according to one of claims 4 to 5, **characterized in that** it represents an average children's skull with respect to one or more of the features selected from the group comprising horizontal frontal curvature, vertical frontal curvature, forehead width, fronto-nasal angle, fronto-occipital diameter, bi-parietal diameter and horizontal radius of curvature of the occipital module.

7. Three-dimensional model according to one of claims 1 to 3, **characterized in that** it is on a percentile curve that is ≤ 10%, based on a normally distributed population of children aged 0-1 years, with respect to one or more of the features selected from the group comprising horizontal frontal curvature, vertical frontal curvature, forehead width, fronto-nasal angle, fronto-occipital diameter, bi-parietal diameter and horizontal radius of curvature of the occipital module.

8. Three-dimensional model according to claim 7, **characterized in that** it is on a percentile curve which is 3% ≤ x% ≤ 10%, preferably 3%, based on a normally distributed population of children aged 0-1 years.

9. Three-dimensional model according to any one of claims 1 - 3, **characterized in that** it is on a percentile curve which is 10% <x% ≤ 30%, based on a normally distributed population of children aged 0-1 years, with respect to one or more of the features selected from the group comprising horizontal frontal curvature, vertical frontal curvature, forehead width, fronto-nasal angle, fronto-occipital diameter, bi-parietal diameter and horizontal radius of curvature of the occipital module.

10. Three-dimensional model according to any one of claims 1 - 3, **characterized in that** it is on a percentile curve that is 30% <x% ≤ 70%, based on a normally distributed population of children aged 0-1 years, with respect to one or more of the features selected from the group comprising horizontal frontal curvature, vertical frontal curvature, forehead width, fronto-nasal angle, fronto-occipital diameter, bi-parietal diameter and horizontal radius of curvature of the occipital module.

11. Three-dimensional model according to claim 10, **characterized in that** it is on a percentile curve which is 45% ≤ x% ≤ 55%, based on a normally distributed population of children aged 0-1 years.

12. Three-dimensional model according to any one of claims 10-11, **characterized in that** it is on a percentile curve which is 50% based on a normally distributed population of children aged 0-1 years.

13. Three-dimensional model according to any one of claims 1 - 3, **characterized in that** it is on a percentile curve that is 55% <x% ≤ 70%, based on a normally distributed population of children aged 0-1 years, with respect to one or more of the features selected from the group comprising horizontal frontal curvature, vertical frontal curvature, forehead width, fronto-nasal angle, fronto-occipital diameter, bi-parietal diameter and horizontal radius of curvature of the occipital module.

14. Three-dimensional model according to any one of claims 1 - 3, **characterized in that** it is on a percentile curve that is 70% <x% ≤ 90%, based on a normally distributed population of children aged 0-1 years, with respect to one or more of the features selected from the group comprising horizontal frontal curvature, vertical frontal curvature, forehead width, fronto-nasal angle, fronto-occipital diameter, bi-parietal diameter and horizontal radius of curvature of the occipital module.

15. Three-dimensional model according to any one of claims 1 - 3, **characterized in that** it is on a percentile curve that is 90% <x% ≤ 97%, based on a normally distributed population of children aged 0-1 years, with respect to one or more of the features selected from the group comprising horizontal frontal curvature, vertical frontal curvature, forehead width, fronto-nasal angle, fronto-occipital diameter, bi-parietal diameter and horizontal radius of curvature of the occipital module.

16. Three-dimensional model according to claim 15, **characterized in that** it is on a percentile curve which is 97%, based on a normally distributed population of children aged 0-1 years.

17. Three-dimensional model of a human child skull in a life-size according to one of the claims 7-16, **characterized in that** it is on the respective percentile curve in the life range of children aged 12-20 weeks.

18. Three-dimensional model of a human child skull in a life-size according to one of claims 7-16, **characterized in that** it is on the respective percentile curve in the life range of children aged 24-32 weeks.

19. Three-dimensional model according to one of claims 1 - 18, **characterized in that** it is reduced regarding its outer dimensions of the bone strength of a human child's skull accordingly by 1-5 mm.

20. Three-dimensional model according to one of the claims 1-19, **characterized in that** it comprises a mold shell (1) which detachably rests on an inner support element (2).

21. Three-dimensional model according to claim 20, **characterized in that** the mold shell (1) comprises the frontal part (8), the parietal part and the occipital main part which are connected to one another.

22. Three-dimensional model according to one of Claims 20-21, **characterized in that** the inner support element (2) is a mount or frame on which the mold shell (1) rests.

23. Three-dimensional model according to one of Claims 20-22, **characterized in that** the outer surface of the mold shell contains recesses (3) for receiving stabilizing elements (4).

24. Three-dimensional model according to Claim 23, **characterized in that** the recesses (3) are openings in the mold shell, preferably strip-shaped and/or point-shaped openings.

25. Three-dimensional model according to one of Claims 23-24, **characterized in that** the recesses (3) are arranged on the outer surface of the mold shell at regular intervals.

26. Three-dimensional model according to one of claims 20-24, **characterized in that** the mold shell has recesses (3) which enable a connection between stabilizing elements, which are applied onto the outer surface of the mold shell and / or into the recesses (3), with bone plates (10) that are also applied to the mold shell, wherein it is preferred that the connection takes place by screwing or riveting.

27. Three-dimensional model according to claim 26, **characterized in that** the recesses (3) according to claim 26 are wholly or partly identical with the recesses (3) according to one of claims 23-25.

28. A kit comprising at least one, preferably at least two, preferably at least three, different models according to one of claims 1-27.

29. Kit according to claim 28, comprising five, preferably six different models according to one of the claims 1-27.

30. Kit according to any one of claims 28-29, further comprising one or more members selected from the group:
- resorbable stabilizing elements (4), in particular plates or strips which are suitable for stabilizing skull bone fragments,
- resorbable screws and / or rivets for fixing skull bone fragments on the stabilizing elements,
- tool for inserting and / or removing the screws or rivets, in particular screwdrivers and screw remover.

31. Kit according to claim 30, **characterized in that** the resorbable stabilizing elements (4) are moldable in a heated state.

32. Kit according to claim 31, **characterized in that** the resorbable stabilizing elements (4) are resorbable lactic acid plates or strips.

33. Kit according to any one of claims 30-32, **characterized in that** the plates or strips have a physiologically acceptable thickness, width and length, wherein a kit comprises uniformly sized plates and strips or plates and strips of different sizes.

34. Method of planning and preparing an operation of a patient with cranial synostosis comprising the steps of:
- Detection and analysis of skull shape features in a group of healthy patients with respect to cranial growth,
- Calculation of a virtual child skull, based on the detected and analysed skull shape features,
- Conversion of the calculated child skull into a stereolithographic model according to one of claims 1-27.

35. Method according to claim 34, **characterized in that** the child's skull is an average child's skull.

36. Method according to claim 35, **characterized in that** in addition to the average child skull, a child skull with maximum shape deviation of individual features, such as head length, - width, frontal width, or -curvature, is calculated and/or converted.

37. Method according to one of Claims 34-36, **characterized in that** the skull-shaped features are selected from horizontal frontal curvature, vertical frontal curvature, forehead width, fronto-nasal angle, fronto-occipital diameter, bi-parietal diameter, horizontal radius of curvature of the occipital module, head length, head width, frontal curvature.

38. Method according to one of the claims 34-37, **characterized in that** detection and analysis of skull-shaped features takes place in a group of patients aged 12-20 weeks and 24-32 weeks, which is healthy with respect to the cranial growth.

39. Method according to one of claims 34-38, **characterized in that** all measured values are documented in percentile curves.

40. Method according to one of Claims 34-39, **characterized in that** a virtual average skull is calculated for children at the age of 4 or 8 months, as well as one skull each with a maximum shape deviation of individual skull shape features.

41. Method according to one of Claims 36-40, **characterized in that** the average skull and the skull with maximum shape deviation are converted into 5-8 stereolithographic models according to one of Claims 1-27.

42. Method according to any one of claims 34-41 comprising the steps of:
- Providing skull reconstructions of cranial growth-healthy patients aged 12-20 weeks and 24-32 weeks,
- Detection and analysis of typical features of the skull, such as horizontal frontal curvature, vertical frontal curvature, frontal width, fronto-nasal angle, fronto-occipital diameter, bi-parietal diameter, horizontal radius of curvature of occipital module,
- Documentation of all measured values in percentiles
- Calculation of a virtual average skull for children aged 4 or 8 months and/or one skull with a maximum shape deviation of individual features, such as head length, head width, frontal width or frontal curvature,
- Translating the average skull and/or the skull/skulls with maximum shape deviation into 5-8 stereolithographic sterilisable models according to one of claims 1-27.

43. The method of claim 42, further comprising the steps of:
- Selection of the stereolithographic model most similar to the patient,
- Sterile provision of the model in the operating room.

## Revendications

1. Modèle stérilisable à trois dimensions d'un crâne humain grandeur nature, comprenant une partie sommitale et une partie principale arrière, **caractérisé en ce qu'**il comprend une partie frontale (8) et également une partie de base (7), sur laquelle la partie frontale (8), la partie sommitale et la partie principale arrière peuvent être agencées de manière amovible, **caractérisé en outre en ce que** le modèle est un modèle de crâne infantile.

2. Modèle à trois dimensions selon la revendication 1, **caractérisé en ce qu'**au moins deux parties, choisies parmi la partie frontale (8), la partie sommitale et la partie principale arrière sont reliées l'une à l'autre.

3. Modèle à trois dimensions selon l'une des revendications 1 à 2, **caractérisé en ce que** la partie sommitale et la partie principale arrière sont reliées ensemble pour former une partie principale arrière et sommitale (9).

4. Modèle à trois dimensions selon l'une quelconque des revendications, **caractérisé en ce qu'**il représente un crâne infantile moyen, dans lequel la moyenne se réfère à une population normalement répartie d'enfants âgés de 12 à 20 semaines.

5. Modèle à trois dimensions selon l'une quelconque des revendications précédentes, **caractérisé en ce qu'**il représente un modèle de crâne infantile moyen, dans lequel la moyenne se réfère à une population normalement répartie d'enfants âgés de 24 à 32 semaines.

6. Modèle à trois dimensions selon l'une quelconque des revendications 4 à 5, **caractérisé en ce qu'**il représente un crâne infantile moyen concernant une ou plusieurs des caractéristiques choisies dans le groupe comprenant la convexité frontale horizontale, convexité frontale verticale, largeur frontale, angle fronto-nasal, diamètre fronto-occipital, diamètre bipariétal, et rayon de courbure horizontale du module occipital.

7. Modèle à trois dimensions selon l'une quelconque des revendications 1 à 3, **caractérisé en ce qu'**il se trouve sur une courbe percentile qui est <= 10%, par rapport à une population normalement répartie d'enfants entre 0 et 1 an, en ce qui concerne une ou plusieurs des caractéristiques choisies dans le groupe comprenant la convexité frontale horizontale, convexité frontale verticale, largeur frontale, angle fronto-nasal, diamètre fronto-occipital, diamètre bipariétal, et rayon de courbure horizontal du module occipital.

8. Modèle à trois dimensions selon la revendication 7, **caractérisé en ce qu'**il se trouve sur une courbe percentile qui est 3% < = à x% < = à 10 %, de préférence 3%, par rapport à une population normalement répartie d'enfants entre 0 et 1 an.

9. Modèle à trois dimensions selon l'une quelconque des revendications 1 à 3, **caractérisé en ce qu'**il se trouve sur une courbe percentile qui est 10% < x% < = 30%, par rapport à une population normalement répartie d'enfants entre 0 et 1 an, en ce qui concerne une ou plusieurs des caractéristiques choisies dans le groupe comprenant la convexité frontale horizontale, convexité frontale verticale, largeur frontale, angle fronto-nasal, diamètre fronto-occipital, diamètre bipariétal, rayon de courbure horizontale du module occipital.

10. Modèle à trois dimensions selon l'une quelconque des revendications 1 à 3, **caractérisé en ce qu'**il se trouve sur une courbe percentile, qui est 30% < x% < = 70 %, par rapport à une population normalement répartie d'enfants entre 0 et 1 an, en ce qui concerne une ou plusieurs des caractéristiques choisies dans le groupe comprenant la convexité frontale horizontale, convexité frontale verticale, largeur frontale, angle fronto-nasal, diamètre fronto-occipital, diamètre bipariétal, rayon de courbure horizontale du module occipital.

11. Modèle à trois dimensions selon la revendication 10, **caractérisé en ce qu'**il se trouve sur une courbe percentile, qui est 45% < = x% < = 55 %, par rapport à une population normalement répartie d'enfants de 0 à 1 an.

12. Modèle à trois dimensions selon l'une quelconque des revendications 10 à 11, **caractérisé en ce qu'**il se trouve sur une courbe percentile qui est 50%, par rapport à une population normalement répartie d'enfants entre 0 et 1 an.

13. Modèle à trois dimensions selon l'une quelconque des revendications 1 à 3, **caractérisé en ce qu'**il se trouve sur une courbe percentile, qui est 55% < à x% < = 70 %, par rapport à une population normalement répartie d'enfants entre 0 et 1 an, en ce qui concerne une ou plusieurs des caractéristiques choisies dans le groupe comprenant la convexité frontale horizontale, convexité frontale verticale, largeur frontale, angle fronto-nasal, diamètre fronto-occipital, diamètre bipariétal, rayon de courbure horizontale du module occipital.

14. Modèle à trois dimensions selon l'une quelconque des revendications 1 à 3, **caractérisé en ce qu'**il se trouve sur une courbe percentile, qui est 70% < x% < = 90 %, par rapport à une population normalement répartie d'enfants entre 0 et 1 an, en ce qui concerne une ou plusieurs des caractéristiques choisies dans le groupe comprenant la convexité frontale horizontale, convexité frontale verticale, largeur frontale, angle fronto-nasal, diamètre fronto-occipital, diamètre bipariétal, rayon de courbure horizontale du module occipital.

15. Modèle à trois dimensions selon l'une quelconque des revendications 1 à 3, **caractérisé en ce qu'**il se trouve sur une courbe percentile, qui est 90% < x% < = 97 %, par rapport à une population normalement répartie d'enfants entre 0 et 1 an, en ce qui concerne une ou plusieurs des caractéristiques choisies dans le groupe comprenant la convexité frontale horizontale, convexité frontale verticale, largeur frontale, angle fronto-nasal, diamètre fronto-occipital, diamètre bipariétal, rayon de courbure horizontale du module occipital.

16. Modèle à trois dimensions selon la revendication 15, **caractérisé en ce qu'**il se trouve sur une courbe percentile, qui est 97%, par rapport à une population normalement répartie d'enfants entre 0 et 1 an.

17. Modèle à trois dimensions d'un crâne humain infantile grandeur nature selon l'une quelconque des revendications 7 à 16, **caractérisé en ce qu'**il se trouve sur la courbe percentile respective dans la tranche d'âge d'enfants âgés de 12 à 20 semaines.

18. Modèle à trois dimensions d'un crâne humain infantile grandeur nature selon l'une quelconque des revendications 7 à 16, **caractérisé en ce qu'**il se trouve sur la courbe percentile correspondante dans la tranche d'âge d'enfants entre 24 et 32 semaines.

19. Modèle à trois dimensions selon l'une quelconque des revendications 1 à 18, **caractérisé en ce qu'**il est réduit dans sa dimension la plus externe des épaisseurs d'os de 1 à 5 mm par rapport à un crâne infantile humain.

20. Modèle à trois dimensions selon l'une quelconque des revendications 1 à 19, **caractérisé en ce qu'**il comprend une coque de moule (1) qui repose de manière amovible sur/contre un élément de support interne (2).

21. Modèle à trois dimensions selon la revendication 20, **caractérisé en ce que** la coque de moule (1) comprend la partie frontale (8), la partie sommitale et la partie principale arrière, qui sont reliées l'une l'autre.

22. Modèle à trois dimensions selon l'une quelconque des revendications 20 à 21, **caractérisé en ce que** l'élément de support interne (2) est un tréteau ou un cadre, sur lequel la coque de moule (1) repose.

23. Modèle à trois dimensions selon l'une quelconque des revendications 20 à 22, **caractérisé en ce que** la surface la plus extérieure de la coque de moule comprend des ouvertures (3) pour recevoir des éléments de stabilisation (4).

24. Modèle à trois dimensions selon la revendication 23, **caractérisé en ce que** les ouvertures (3) sont des ouvertures dans la coque de moule, en particulier ou de préférence des ouvertures formant des bandes et/ou des points.

25. Modèle à trois dimensions selon l'une quelconque des revendications 23 à 24, **caractérisé en ce que** les ouvertures (3) sont agencées sur la surface la plus externe de la coque de moule et sont espacées de manière régulière.

26. Modèle à trois dimensions selon l'une quelconque des revendications 20 à 24, **caractérisé en ce que** la coque de moule comprend des ouvertures ou cavités (3) qui rendent possible une liaison d'éléments de stabilisation rapportée dans les ouvertures (3) et/ou rapportés sur la surface la plus externe de la coque de moule, avec des plaques osseuses (10) également placées depuis l'extérieur sur la coque de moule, dans lequel il est préféré que les liaisons soient faites au moyen de vissage ou de rivetage.

27. Modèle à trois dimensions selon la revendication 26, **caractérisé en ce que** les ouvertures (3) selon la revendication 26 sont identiques en totalité ou en partie avec les ouvertures (3) selon l'une des revendications 23 à 25.

28. Kit comprenant au moins 1, plus particulièrement au moins 2, de préférence au moins 3 modèles différents selon l'une quelconque des revendications 1 à 27.

29. Kit selon la revendication 28, comprenant 5, de préférence 6 modèles différents selon l'une quelconque des revendications 1 à 27.

30. Kit selon l'une quelconque des revendications 28 à 29, comprenant en outre une ou plusieurs parties choisies dans le groupe constitué par :
des éléments de stabilisation résorbables (4), en particulier des plaques ou des bandes, qui sont prévues pour stabiliser des fragments osseux de crâne,
des vis résorbables et/ou des rivets résorbables pour fixer des fragments osseux de crâne sur des éléments de stabilisation,
un outil pour introduire et/ou retirer des vis ou des rivets, en particulier un tournevis et un tournevis pour retirer les vis ou rivets.

31. Kit selon la revendication 30, **caractérisé en ce que** les éléments de stabilisation résorbables (4) peuvent être formés dans un état chauffé.

32. Kit selon la revendication 31, **caractérisé en ce que** les éléments de stabilisation résorbables (4) sont des bandes ou des plaques d'acide lactiques résorbables.

33. Kit selon l'une quelconque des revendications 30 à 32, **caractérisé en ce que** les plaques ou bandes comprennent une longueur, largeur et épaisseur physiologiquement acceptable, dans lequel un kit comprend des plaques et bandes de taille unitaire ou des plaques et bandes de taille différente.

34. Procédé pour planifier et préparer une opération d'un patient avec une synostose crâniale comprenant les étapes suivantes :
détermination et analyse de caractéristiques de forme crânienne dans un groupe de patients sains en ce qui concerne la croissance crânienne,
calcul d'un crâne infantile virtuel, sur la base des caractéristiques de forme crânienne déterminées et analysées,
transposition du crâne infantile calculée dans un modèle stéréo lithographique selon l'une quelconque des revendications 1 à 27.

35. Procédé selon la revendication 34, **caractérisé en ce que** le crâne infantile est un crâne infantile moyen.

36. Procédé selon la revendication 35, **caractérisé en ce que**, en plus du crâne infantile moyen, un crâne infantile est calculé et/ou transposé avec un variation maximale de caractéristiques individuelles, telles que la longueur de tête, la largeur de tête, la largeur frontale, ou la convexité frontale.

37. Procédé selon l'une quelconque des revendications 34 à 36, **caractérisé en ce que** les caractéristiques de forme crânienne sont choisies parmi la convexité frontale horizontale, convexité frontale verticale, largeur frontale, angle fronto nasal, diamètre front occipital, diamètre bipariétal, rayon de courbure horizontale du module occipital, longueur de tête, largeur de tête, convexité frontale.

38. Procédé selon l'une quelconque des revendications 34 à 37, **caractérisé en ce que** la détermination et l'analyse de caractéristiques de forme crânienne a lieu dans un groupe sain en ce qui concerne la croissance crânienne de patients d'âge compris entre 12-20 semaines et 24-32 semaines.

39. Procédé selon l'une quelconque des revendications 34 à 38, **caractérisé en ce que** toutes les valeurs de mesure sont documentées dans des courbes percentiles.

40. Procédé selon l'une quelconque des revendications 34 à 39, **caractérisé en ce qu'**un crâne moyen virtuel est calculé pour des enfants âgés de 4 mois, en particulier 8 mois, ainsi que respectivement un crâne avec un écart de forme maximale/une variation de forme maximale de caractéristiques de forme crânienne individuelle.

41. Procédé selon l'une quelconque des revendications 36 à 40, **caractérisé en ce que** le crâne moyen et les crânes avec des variations de forme maximale sont transposés dans des modèles stéréo lithographique 5-8 selon l'une quelconque des revendications 1 à 27.

42. Procédé selon l'une quelconque des revendications 34 à 41, comprenant les étapes suivantes :
préparation de reconstruction crânienne de patients sains en ce qui concerne la croissance crânienne dans la tranche d'âge entre 12-20 semaines et 24-32 semaines,
détermination et analyse de caractéristique de forme crânienne typique telle que la convexité frontale horizontale, convexité frontale verticale, largeur frontale, angle fronto nasal, diamètre fronto occipital, diamètre bipariétal, rayon de courbure horizontale du module occipital,
documentation de toutes les valeurs de mesure dans des courbes percentiles,
calcul d'un crâne moyen virtuel d'enfant dans la tranche d'âge 4 en particulier 8 mois et/ou respectivement d'un crâne avec des écarts de forme maximaux de caractéristiques individuelles, tels que la longueur de tête, la largeur de tête, la largeur frontale ou la convexité frontale,
transposition du crâne moyen et/ou des crânes/du crâne avec les variations de forme maximale dans des modèles stérilisables stéréo lithographiques 5-8 selon l'une quelconque des revendications 1 à 27.

43. Procédé selon la revendication 42, comprenant en outre les étapes suivantes :
choix du modèle stéréo lithographique le plus proche après comparaison avec le patient, et
préparation stérile du modèle pour une salle d'opération.
